# EUROPEAN PATENT SPECIFICATION

(11) **EP 2 908 664 B1**
(45) Date of publication and mention of the grant of the patent: **26.12.2018**
(21) Application number: 13846579.4
(22) Date of filing: 16.10.2013
(51) Int. Cl.: A23L 33/10, A23L 7/10, A23L 7/152, A23L 11/10, A23L 33/105, A23L 33/15, A23L 33/16, A23L 33/175

(54) **A NOVEL RAW MATERIAL FOR FUNCTIONAL FOODS AND A PROCESS FOR THE PREPARATION THEREOF**
NEUARTIGER ROHSTOFF FÜR FUNKTIONALE LEBENSMITTEL UND VERFAHREN ZUR HERSTELLUNG DAVON
NOUVELLE MATIÈRE PREMIÈRE POUR DES ALIMENTS FONCTIONNELS ET LEUR PROCÉDÉ DE PRÉPARATION

(30) Priority: 16.10.2012 HU P1200592
(43) Date of publication of application: 26.08.2015
(73) Proprietor: FITOREX KFT., 1037 Budapest (HU)
(72) Inventor: SZILBEREKY, Jeno, H-1015 Budapest (HU); JEDNÁKOVITS, Andrea, H-2000 Szentendre (HU); SALGÓ, András, H-1025 Budapest (HU); BARLA SZABÓ, Gábor, H-2330 Dunaharaszti (HU); SZABADOS, László, H-8518 Kemenesszentpéter (HU)
(74) Representative: Machytka-Frank, Daisy
(86) International application number: PCT/HU2013/000100
(87) International publication number: WO 2014/060784

(56) References cited:
- WO-A1-2004/096250
- CN-A- 1 947 582
- RU-C1- 2 136 367
- RU-C1- 2 136 367
- RU-C1- 2 142 721
- RU-C2- 2 187 945
- RU-C2- 2 187 945
- US-A- 6 129 937
- US-A1- 2003 059 516
- US-A1- 2003 059 516
- US-A1- 2005 147 731
- US-A1- 2005 147 731
- SUMIKO NAKAMURA ET AL: "Palatable and Bio-Functional Wheat/Rice Products Developed from Pre-Germinated Brown Rice of Super-Hard Cultivar EM10", BIOSCIENCE BIOTECHNOLOGY BIOCHEMISTRY., vol. 74, no. 6, 23 June 2010 (2010-06-23), pages 1164-1172, XP055286996, TOKYO, JAPAN ISSN: 0916-8451, DOI: 10.1271/bbb.90850

## Description

The present invention relates to a process for the preparation of a novel raw material for functional foods containing bioactive material/materials, furthermore, the product of the novel preparation process and its use.

Further to the fact that cereals, leguminous foods or seeds usable for any kind of nutritional purpose may be very different in their sustenance, it is known that the use of said cereals, leguminous foods or seeds in the everyday nutrition depends on a number of factors. Their application may be determined, among others, by the local eating and cultural conventions, their prevalence in the different parts of the world, and the climatic circumstances, as well. Thus, the composition and application of these nutritional basic materials shows a very different picture in the different parts of the world.

It is also known that the effectiveness of the nutritional and eating awareness is hindered by the fact that the components, nutrients ensuring the beneficial physiological, biological, and dietetical values are present in limited amounts in the cereals, leguminous foods or seeds used for consumption, as well as in the compositions, food and meals made from them.

Accordingly, by the consumption of these, the targeted beneficial physiological, biological, and dietetical effect usually can hardly be ensured or only sub-optimally or ineffectively.

Furthermore, it is also known that amongst the direct and indirect causes of diseases there are fundamental eating problems based on the improperly selected foods, their incautious or misinformed use, the established bad habits, inappropriate traditions. One way of the advantageous change is the development of foods that, based on their content (macro and micro components) and biological conversion, possess an additional medical effect or a specific health supporting effect. Their composition may be elaborated on the basis of the newest scientific results and modern nutritional knowledge, and using general or specific operations, technologies in view of preserving or improving the health of humans. The generic term for products possessing such characteristics is "functional foods".

Known solutions according to the literature were targeted to achieve the physiological, biological, dietetical and/or food processing technological benefit with nutritionally useful seeds, cereals and leguminous foods by various methods, changing the quantity of the content of bioactive materials, preferably by the increase of them.

According to the solutions of the state of the art, the change of the content of the seeds and the modification of the beneficial application values of the various food products prepared from the seeds with the new composition was achieved by various methods. Accordingly, the following methods may be applied: a) cross breeding of the seeds, genetic modification of the seeds, modification of the production methods of the seeds, classical and specific processing operations (such as, e.g. grinding, stirring), b) germinating of the seeds, c) germinating of the seeds followed by fermentation, d) germinating the seeds followed by food-industrial change, processing (such as meat-industrial products, soy-milk, etc.)

As the most usual solution, the beneficial biological, medical effect is achieved by the germination of the cereals and leguminous seeds, and in the course of the germination process the composition of the seed advantageously changes in terms of the concentration of certain bioactive materials.

Such a method was disclosed by US 2008/0274249 patent application, wherein maize, beans, soy-beans, lentils, wheat and barley is germinated, and in this way a more substantial product is produced with higher nutritional value. The review of Nakamura S. et al. (Biosci. Biotechnol. Biochem. 74 (6) 90850-1-9, 2010) discloses the beneficial utilization of germinated brown rice and wheat in bakery and pastry production, wherein, among others, the increase of the content of gamma amino butyric acid (GABA) and the beneficial taste effect of the change of the proteins are underlined.

WO 2004/096250 international publication document discloses that the nutritional values of a soybean advantageously changes during germination, among others phosphatidyl-inositol-kinase enzyme, vitamins C and B complex are synthetised, therefore it is useful for controlling the blood sugar level. The method disclosed by the cited document comprises the following steps: a) soaking raw soybean, b) germinating the seeds, c) drying the germinated seeds and grinding the dried seeds into fine powder. The cited document discloses that the early germinated soybean seeds become enriched with phytochemicals like phosphatidyl inositol 3 kinase, vitamins and dehydrogenases. In contrast to the process according to the present invention, the cited document does not disclose the treatment of the seeds and their sprouts in an aqueous solution or suspension or emulsion of biologically active materials. The beneficial role of soy isoflavonoids (daidsein, genistein), whose content beneficially changes during germination, at vascular events caused by diabetes is disclosed by Xu Sz. et al. (Endocrinology 2009 Jul15,47(2);167-75. E-pub 2009 Apr.22.). A specific germination method useful on an industrial scale with achieving higher genistein content is disclosed by WO2010/055360 international patent application.

Nakamura et al. (Bioscience, Biotechnology and Biochemistry 2010 June 23, 74(6); 1164-1172) discloses the production of high-quality and biofunctional wheat/rice bread and wheat/rice noodles. The disclosed method comprises the pre-germination of the rice component before the preparation of bread/noodle. This solution results in advantageous internal changes in the rice, and a better quality bread (that is, it has better taste and is softer) can be produced from the obtained rice flour. However, the cited document is silent about the process for the preparation of a functional food base material containing any bioactive materials.

Germination methods for unpolished and brown rice are disclosed in KR 20070101926, KR 20070101824 and CN 101406273 patent applications, to produce rice rich in selenium and germanium.

Processes are disclosed in RU 2155513 and RU 2288583 Russian patent applications, wherein in the course of germination of chickpea, a product is achieved, which possesses the effects of preventing atherosclerosis and improving the resistance of the organism through the immune system. By using this product in meat products and bakery products, nutritional compositions with advantageous physiological effects can be produced.

The change of the composition during the germination of oats is disclosed by CN 101507504 patent document. According to this document, the GABA content of oats increases to 10-40 fold of the original value, the carbohydrate content changes, the amount of the mineral components and vitamins increases and a few functional features of oats changes advantageously during germination.

Similar results are disclosed in CN 101366482 patent description in connection with the germination of wheat.

Various germination techniques of brown rice can be found in a number of patent descriptions, e.g. in CN 101536666 patent application and CN 101461540 patent application, according to which a brown rice product with high GABA and flavonoid content is achieved. According to WO2009/110612 international patent application thanks to the new materials generated during the germination of brown rice, the product is used for the prophylaxis of diabetic neuropathy. CN 101396126 patent application discloses the kidney-fuction improving effect of the pasta prepared from unpolished germinated rice, which contains elevated amounts of GABA and flavonoids.

The production of a more tasteful, and, due to its nutritional content, a healthier soy-milk from germinated soy is disclosed in e.g. CN 1934969 patent application and US 2010/0119689 publication document, furthermore, in CN 101642220 patent application, wherein beside the known nutritional benefits, soy-milk beverages containing Ca, Mg and Fe are disclosed. Functional foods made from germinated soy are disclosed in EP 1363507. With the same germinated soy as the starting material, and by further fermentation processes 10 to 40 per cent higher isoflavonoid levels (KR 100823896 patent document), higher amino acid levels (KR 100816558 patent document), and bird flu preventing effects (KR 100796303 patent document) are achieved.

A novel plant breeding method is disclosed in CN 101080988 patent specification, in connection with the production of green soy containing higher amounts of iodine.

US 2007/0292541 publication document and 6,028,251 patent description disclose similar solutions. The seeds are soaked in solutions containing vitamins B in the first case, and various ions in the second case, with the view of germination. Due to the germination, they achieved in both cases that the sprouts and the germs are enriched in vitamins B and in the ions of the electrolyte solution, respectively.

US 2003/0059516 publication document discloses a method, wherein the grown germ is cooked in an alkaline solution, and the product is used for the flavouring of foods.

The fundamental limitation of all these known processes, and thus, the qualitative and quantitative hindrance is due to the fact that the amount of the biologically active materials, which originally exists or generates during the production process in cereals, leguminous foods or seeds suitable for human consumption, due to the nature of said plants, can change or generate only to a limited extent, and thus the desired results and effects become also limited.
It is a further drawback of the known processes that the technological features or beneficial biological effects achieved by the known methods, besides the above-mentioned natural quantitative and qualitative limitations of the raw materials of foods, are eventual, cannot be applied to any kind of chemical components, only to those that can be found in the given plant species, or can be generated in it.

A fundamental drawback of the known processes is that the (limited) new characteristics are restricted to those that, based on the physical and chemical nutritional features of the food material (cereals, leguminous foods and other seeds) already existed or were spontaneously generated.

Summarizing the above, the principal disadvantage of the processes, wherein the starting steps of plant breeding, e.g. the growth of the germ or the sprout, are combined with the incorporation of a limited number of materials that are rare in or absent from the plant (vitamins, mineral ions), is that within the natural biological conditions of the plants, that is, within the physical and temperature conditions of the germ and sprout generation, the amount of the incorporated vitamins B and ions is very limited.

Furthermore, each of the above-mentioned processes aims to the (limited) improvement of the composition of the germ or the sprout, and does not aim to transform the nutritional value of the starting seeds.

In the course of our experiments the object of the present inventors was the preparation of novel, functional raw materials for food, and their processing to foods that are devoid from the natural limitations of the above-discussed methods and raw materials, and the new food products contain arbitrarily selected components, which are considered to be the most suitable for the given functional nutritional goal considering the results of the state of the art.

In particular, our aim was to ensure the biologically, dietetically maximum amount (including, certainly, the most preferred amount, too) of the functional food components in the new functional food starting material and composition taken in, furthermore, the aim was that all relevant materials ensuring the chosen nutritional function would be incorporated irrespectively to the original composition of the raw material.

Another reason for seeking of a novel solution was the fact that the preferred incorporation and consumption of e.g. vitamins and minerals are in their natural form, *via* foods, because of their better bioavailability, as e.g. the antioxidants are in a balanced proportion (in oxidated and reduced forms) in the foods, while this balance is missing from the dietary supplementary compositions. The bioavailability of the incorporated components can thus be significantly improved.

The present invention is based on the two discoveries that by using the optimum biological, dietetical condition (maximum nutrition value) of the seeds from the aspect of the human utilization, and building on said maximum nutrition value, by a treatment with elevated temperature, whose intensity is higher than that of the conventional biological, physical conditions of plant breeding, the seeds possessing the optimal features may incorporate higher amounts of the new materials, thus, as a result of the above-mentioned aspects, novel, valuable food materials can be formed.

In the present description the term nutrition value shall mean the common feature of one kind of food or food basic material characterized by the major components thereof. (Gebhardt, Susan E., and Robin G.. 2002. Nutritive Value of Foods. U.S. Department of Agriculture, Agricultural Research Service, Home and Garden Bulletin 72). The most often measured food components are as follows: calory (intake), protein, water, whole fat content; as a part of it: saturated, unsaturated, and poly-unsaturated fatty acids, cholesterol; carbohydrate, dietary fiber, calcium, iron, potassium, sodium, vitamin A (IU and RE values), thiamine, riboflavin, niacin and vitamin C.

The present invention thus is based on the finding that in the state of maximum nutrition vale of cereals, leguminous foods, and any other plant seeds usable for nutritional purposes, where said state is achieved by a specific germination process, the parallel incorporation of more than one biologically active components into said food materials in higher amounts may be achieved by the cooking of the seeds in the aqueous solution, suspension or emulsion of the active compounds, where said solution, suspension or emulsion has the desired concentration of the active compounds, thus the above-mentioned incorporation is effected. Based on our discovery, the active compounds carrying the function may be incorporated in the selected food material, and their amount can be controlled by the desired modification of the concentration of the solution and the parameters of the process.

In the process according to the present invention the biologically active materials are treated at a temperature, which is bearable by the given material without decomposition, thus, besides the cooking at 100 °C in a suspension or emulsion, a lower temperature treatment may be applied in vacuum.

Accordingly, our invention is a process for the preparation of a new type of functional food basic materials containing bioactive materials, which comprises the activation of the enzymes of the edible plant seeds at a temperature of 10-35 °C for a period of 5-72 hours with oxygen and water until reaching the maximum nutritional value of the seeds, then the seeds thus obtained together with their sprouts are treated in the aqueous solution or suspension or emulsion of physiologically or dietetically advantageous materials between 60-100 °C for a period of 10-180 minutes, and then the thus obtained raw material containing the physiologically important biological materials in this enhanced concentration is separated, dried, optionally chopped and packed.

In the process according to the present invention the seeds of cereals and leguminous plants are used as edible plant seeds. As edible plant seed soy-bean seed, rice, wheat, oats, chickpea or maize, or their germinated forms generated by a method known *per se* may be used.

In the process according to the present invention, as physiologically or dietetically advantageous material a material selected from the following group may be used: ions, trace elements, vitamins, poly-unsaturated fatty acids, L-carnitine, coenzyme Q10, antioxidants, lecithin, gamma orisanol, folic acid, gamma amino butyric acid, koline and others.

Furthermore, the invention relates to a new type of (functional) food basic materials enriched with active materials, which is prepared by the process according to the invention as disclosed above.

Still further, the invention relates to the use of the product according to the invention for the preparation of new, modern, (functional) foodstuff, and products containing them.

By the solution provided according to the present invention a food basic material with arbitrary bioactive material composition can be formed, for arbitrarily selected functional food consuming groups. The solution according to the present invention also makes it possible that, independently from the original composition of the used basic material, the new food basic materials and compositions carrying the given function contain all scientifically approved active materials (vitamins, mineral materials, etc.) in optimal quantity and quality (or, in a justified case, even in a higher concentration) incorporated.

In the formulation of the food basic material according to the present invention the following procedure is accomplished: after the due preparation (cleaning, washing, optionally preconditioning) of the given seed (wheat, soy-bean, rice, maize, oats or other) the internal enzymes of the seed are activated by oxygene and water for a short period of time, until the nutritional value of the seed increases. The thus minimally sprouted beans or seeds are cooked until soft in a solution, suspension or emulsion of the materials that are desired to be incorporated in the seeds (beans), at a temperature close to the boiling point of the solvent, for a period of time depending from the type of the seed. After the removal of the cooking solution and cooling of the product, it is dried from the surface moisture, packed, preserved, and/or processed to a food product.

The optimum amount of the incorporated active materials in the given food basic material is determined separately for each product, following in every case of the active material the freely consumable daily allowed intake as the highest value, which is incorporated in the reasonable daily consumed amount of the food basic material, which is 200-300 g of basic material.
The cardioprotective functional diet elaborated for patients suffering from circulatory disorders and hypertension requires that the protein intake take place from a source devoid of cholesterol, preferably from soy protein, furthermore that the cholesterol level decreasing effect of the high nutritional fiber content should prevail. The foodstuff should be of a composition rich in K , Mg, Ca and poly-unsaturated fatty acids (omega-3, omega-6), furthermore enriched in compounds with antioxidant effect, decreasing the oxidative stress effect, such as e.g. C, E vitamins, beta-carotene, selenium and isoflavonoids (daidsein, genistein). The atherosclerosis is delayed by the suitable folic acid and B₆-B₁₂ vitamin content, furthermore, the intake of Zn, Mn and Cu is also necessary, as these elements are usually absorbed in decreased amounts in patients suffering from cardiac diseases. The appropriate amount of L-carnitine improves the blood supply of the coronary vessels, decreases the occurrence of arrhythmias, and promotes the increase of the performance of the heart. The Q10 coenzyme content of the functional food helps the energy production of the cells of the cardiovascular system, furthermore, decreases the harmful effect of the free radicals.

In the modern society the functional diet of patients suffering from the more and more widespread and already regarded as endemic diabetes requires the daily intake of materials that fundamentally influence the carbohydrate metabolism, such as vitamins B (transformation of sugars into energy), Cr (improvement of the utilization of sugars), Zn (needed for the production of insulin). The soy protein-based diet, which is rich in poly-unsaturated fatty acids having the effect of decreasing the cholesterol level, dietary fibers, and is devoid of carbohydrates is desired. Diabetic people need more daily intake from Ca, Mg and K as compared to the level provided by the average food consumption, however, due to the tenfold higher risk factor of the cardiovascular complication, the daily intake of antioxidants, L-carnitine and coenzyme Q10 is also required.

Osteoporosis affects almost every elderly, but primarily women, who can be targetedly helped by the functional diet for osteoporosis, which is rich in specific ions and vitamins. The Ca and P content of the functional food basic materials and foods according to the invention is indispensable for the building up of the bones, their Mg content is required in an appropriate proportion (Ca/P/Mg 1:1:0,5) for the growth of the bones. The vitamin D content helps, among others, the absorption of Ca and P, but the appropriate Zn, Mn and Cu content of the food is important for the building of the bones. The isoflavonoids and vitamin C (in the formation of the collagen fiber) and vitamin A (in the healthy development of the bones) are all important in the daily diet of the population being at risk of osteoporosis.

Specific functional diet is required by the patients suffering from various gastrointestinal diseases. In this case the stress is primarily on the food with high dietary fiber content, and, if possible on the low-fat diet, consuming functional nutrients, which are rich in folic acid and vitamins C, B₁ and B₃.

The treatment of dementia appearing as a problem of the elderly can be helped by such functional foods, which preferably contain among others higher amounts of antioxidants and omega-3-fatty acid, vitamin B (B6, koline), vitamins E and C, which together enhance the brain function, and help preserve the mental acuity.

Obesity, which is considered to be almost endemic in certain parts of the world, also requires the promotion and formation of the functional diet of the obese. Beside the lowered carbohydrate or carbohydrate-free diet it is reasonable to consume foods made from such basic materials that are of preferred fat-composition (poly-unsaturated fatty acids), high dietary fiber content, which at the same time help the formation of the favourable composition of the blood fats, too. As amongst the obese the appearance of II-type, that is non-insuline dependent diabetes is significant, the patients suffering from obesity may apply the functional foods advantageous for the diabetic people, too.

The appropriate diet of athletes, including people doing fitness and body building activities, has already become a separate science. Although it is various, the functional diet according to the sports nutrition, has in general high protein and fiber content, and is rich in vitamins and minerals. The new functional food basic material should advantageously contain lecithin, furthermore, L-carnitine (which optimizes the perfomance, helps the regeneration of the organism, and thus delays the appearance of exhaustion), and coenzyme Q₁₀, which catalyzes the energy production of the cells of the organism, and furthermore, it must have an elevated content of K, Mg and Ca (K and Mg promote, among others, the effective utilization of the amino acids). The gamma-oryzanol (which is the mixture of plant sterols and ferulic acid) content helps the increase of the muscle mass, it is a strong antioxidant, and lowers the LDL level of the blood.

The vegetarian form of diet needs to be emphasized, which, according to the present invention, may expressedly be helped by the specific vegetarian functional food basic materials and diet of plant origin. It is generally known that in the diet of the vegetarians more minerals and vitamins may be of need. Thus, for them new food basic materials with appropriately set, high Fe, Zn, vitamins B₂, B₁₂, D and A, K, Ca, Mg, folic acid, Q₁₀ and poly-unsaturated fatty acid contents are needed.

Further to the above, the new food basic materials obtainable according to the present invention may be used advantageously with other, arbitrarily selected groups of foodstuff, primarily by the formation of basic materials and ready to consume foods and meals containing the compositions according to their dietetic, physiological need, thus, e.g. without limitation, in the following fields: the health-conscious diet (low cholesterol and/or antioxidant rich and/or high fiber diets, etc.), furthermore, low carbohydrate foods (dry pasta, bakery products, menus based on specific recipes), or e.g. in hospital meals of the diets of the customary 15 to 20 types of diet groups.

The consumption of the functional food basic materials according to the invention, and the foods prepared by the utilization thereof is beneficial for not only the given functional group of patients, but also, provided it can be linked to the disease affecting the given group, in the majority of the cases for the prophylaxis of the disease.

According to the present invention functional food basic materials of various composition can be prepared, e.g. those containing gamma amino butyric acid (GABA), folic acid, choline, gamma-oryzanol and other bioactive materials.

The food basic materials according to the present invention are dried as a whole, in ground or pulpy form, in moist state, or, as needed by the operations known and conventional in the food industry, by mild drying to the desired level, and may be milled to a powder with the desired particle size. The packed, moist basic material may be preserved by known methods. The food basic materials may be consumed in themselves, but any desired functional food may be prepared from them.

The new products according to the present invention, and the compositions made from them, furthermore, the processes according to the present invention will be further described by way of the following examples.

### Example 1

### Soy, containing cardioprotective bioactive materials

Soy-bean (200 g) is washed carefully with water of ambient temperature in a filter. The washing should contact each seed, and lasts for maximum 1 minute. The washed soy is left to stand for a short period of time, while the seed takes the water up. Then the soy-bean is transferred to a vessel of appropriate size, and 500 ml aqueous solution of potassium chloride (2.86 g) magnesium chloride x 6H₂O (4.16 g), calcium chloride x 2H₂O (1.8 g), ascorbic acid (100 mg), beta-carotine (1000 µg), sodium selenite (70 µg), folic acid (200 µg), pantothenic acid (6 mg), furthermore L-carnitine (500 mg) and coenzime Q₁₀(20 mg) is poured on it. The soy in the solution is cooked until soft for 120 minutes at a temperature of 80-100 °C, or at reduced pressure at a steam temperature of 80 °C. Then the solution is removed from the seeds, the surface of the seeds is washed with water, and following a short period of drying time, the product is packed, and if desired, preserved by known methods.

The bioactive material concentration of the product obtained is the following (in the following, the starting material concentrations are indicated in brackets): 153.8 mg (116.0 mg) Ca; 162.9 mg (130.2 mg) Mg; 1212.30 mg (919.6 mg) K; 33.15 mg (6 mg) ascorbic acid; 20.1 µg (15.9 µg) selenium; 310.02 µg beta-carotine; 247 µg folic acid; 87.08 mg L-carnitine and 6.9 mg Q₁₀/100 g product.

### Example 2

### Soy, containing cardioprotective bioactive materials

a) The process according to Example 1 is followed, except that the seeds are germinated in a method standardized and known *per se,* but using preconditioning at a temperature between 25-27 °C until they reach the germ size of a couple of mm-s, then, depending on the variety of the seed, the seed germinated for 36-48 hours (together with the germs) is cooked in the aqueous solution for 120 minutes, at 100 °C until soft.
b) Every aspect of the process variety a) is followed, except the cooking is done at reduced pressure and at 40-60 °C steam temperature;
c) Every aspect of the process variety b) is followed, except the cooking is done at reduced pressure and at 40-60 °C steam temperature, and in an inert atmosphere (nitrogen);

The bioactive material concentration of the soy product (YASO) obtained is the following: 227.3 mg (98.3 mg) Ca; 195.9 mg (76.9 mg) Mg; 484.5 mg (344.5 mg) K; 36.12 mg (6 mg) ascorbic acid; 22.1 µg (8.6 µg) selenium; 271 µg beta-carotine; 263 µg folic acid; 285.9 mg L-carnitine and 10.5 mg Q₁₀/100 g product.

### Example 3

The processes according to Examples 1 or 2 are followed, except that as starting material rice or wheat or oats or maize is used in place of soy, and the germination is done, depending on the species of the seed, until 2 to 8 mm germ length in line with Example 2.

The bioactive material concentration of the wheat product obtained by Example 1 is the following: 34.29 mg (26.6 mg) Ca; 100.70 mg (87.0 mg) Mg; 370.1 mg (339.2 mg) K; 34.12 mg ascorbic acid; 69.2 µg (49.5 µg) selenium; 240 µg (91 IU) beta-carotine; 116 mg L-carnitine and 6 mg Q₁₀ /100 g product.

The bioactive material concentration of the rice product germinated for a short period of time by the standardized method according to Example 2 is the following: 37.29 mg (13.1 mg) Ca; 91.70 mg (54.1 mg) Mg; 152.30 mg (63.2 mg) K: 34.12 mg ascorbic acid; 18 µg selenium; 290 µg beta-carotine; 78 µg (21 µg) folic acid; 140 mg L-carnitine and 6 mg Q₁₀/100 g product.

### Example 4

### Soy with advantageous composition for diabetic patients (functional food basic material)

The processes according to Examples 1 or 2 are followed in every aspect, except that as cooking solution the following is applied: the aqueous solution of potassium chloride (2.86 g) magnesium chloride x 6H₂O (4.16 g), calcium chloride x 2H₂O (1.8 g), chromous sulphate x 6H₂O (0.3 g), zinc sulphate x 7H₂O (0.24 g), beta-carotine (1000 µg), sodium selenite (70 µg), folic acid (200 µg), pantothenic acid (6 mg), furthermore L-carnitine (500 mg) and coenzime Q₁₀(20 mg).

The bioactive material concentration of the soy product (YASO) produced in line with the standardized method of Example 2, with reduced carbohydrate content, germinated for short period of time: 486.6 mg (344.5 mg) K; 198.2 mg (76.9 mg) Mg; 228.6 mg (98.3 mg) Ca; 1.96 mg Cr; 11.86 mg (2.71 mg) Zn; 21.1 µg (8.2 µg) selenium; 255 µg folic acid; 325.02 µg beta-carotene; 2.4 mg (0.8 mg) pantothenic acid; 122.40 mg L-carnitine és 6.3 mg Q₁₀/100 g product.

### Example 5

### Functional food basic material with a composition beneficial for athletes

The processes according to Examples 1, 2 or 3 are followed, except that as cooking solution 300 ml aqueous solution of potassium chloride (2.86 g) magnesium chloride x 6H₂O (4.16 g), calcium chloride x 2H₂O (1.8 g), L-carnitine (600 mg), lecithine (2.5 g) and coenzyme Q₁₀ (50 mg) is cooked at 100 °C.

### Products:

The incorporated bioactive material concentration of the soy basic material according to the example is as follows: 237.3 mg (96.3 mg) Ca; 179.4 mg (73.9 mg) Mg; 472.5 mg (340.5 mg) K; 289.9 mg L-carnitine; 7.8 mg Q₁₀ és 380 mg lecithin /100 g product.

The incorporated bioactive material concentration of the rice basic material obtained according to Example 2 is as follows: 39.38 mg (13.9 mg) Ca; 98.70 mg (51.1 mg) Mg; 159.30 mg (62.2 mg) K; 175 mg L-carnitine; 220 mg lecithin and 18.2 mg coenzyme Q₁₀/100g product.

The incorporated bioactive material concentration of the wheat basic material obtained according to Example 2 is as follows: 32.41 mg (11.9 mg) Ca; 77.70 mg (39.1 mg) Mg; 130.3 mg (50.2 mg) K; 188 mg L-carnitine; 231 mg lecithin and 16.2 mg coenzyme Q₁₀ /100 g product.

### Example 6

### Functional food basic material with a composition beneficial for vegetarians

a) In every aspect Examples 1, 2 and 3 are followed, except that as cooking solution 300 ml aqueous solution of zinc sulphate (0.24 g), piridoxine HCI (28 mg), vitamin B12 (2.0 mg), beta-carotene (1000 µg), potassium chloride (2.86 g), magnesium chloride x 6H₂O (4.16 g), calcium chloride x 2H₂O (1.8 g), folic acid (200 µg) and coenzyme Q₁₀ (20 mg) is used at 100 °C.
b) Every aspect of the process variety a) is followed, except the cooking is done at reduced pressure and at 50-80 °C steam temperature;

### Products:

The incorporated bioactive material concentration of the soy basic material according to Example 2 is as follows: 481.5 mg (342.5 mg) K; 231.2 mg (96.3 mg) Ca; 181.4 mg (73.9 mg) Mg; 10.65 mg (2.1 mg) Zn; 22.34 mg (1.6 mg) piridoxine; 0,9 µg B12; 7.3 mg Q₁₀ and 251 µg folic acid /100 g product.

The incorporated bioactive material concentration of the rice basic material according to Example 2 is as follows: 147.3 mg (61.2 mg) K; 37.57 mg (13.9 mg) Ca; 91.70 mg (52.1 mg) Mg; 5.89 mg (0.9 mg) Zn; 2.2 mg piridoxine; 0.7 µg B12; 310 µg beta-carotene; 74 µg (20 µg) folic acid and 12.2 mg coenzyme Q₁₀/100 g product.

The wheat basic material obtained according to Example 2: 33.40 mg (10.9 mg) Ca; 75.74 mg (36.1 mg) Mg; 135.4 mg (47.2 mg) K; 2.4 mg piridoxine; 0.8 µg B12; 302 µg beta-carotene és 15.2 mg coenzyme Q₁₀/100 g product.

### Example 7

### Functional food basic material beneficial for patients suffering from dementia

5 kg appropriately preconditioned soy with reduced carbohydrate content (YASO) soaked for a short period of time, germinated up to 5-10 mm germ length is cooked in 7.5 I solution of 7 g piridoxine HCI, 11 g koline chloride, 350 mg alpha-tocoferol acetate, 2.2 g ascorbic acid at reduced pressure, at maximum 40-60 °C steam temperature until soft. The germinated, cooked soy is then filtered in its warm state, and the residue of the solution is washed down from the surface of the seeds with water of drinking water quality, and cooled to room temperature. The product thus obtained is processed to a foodstuff or packed as desired, and preserved.

The incorporated bioactive material concentration of the soy product germinated for a short period of time according to Example is as follows: 25.3 mg (0.4 mg) vitamin B₆; 124 mg (23 mg) koline; 5.3 mg (3.2 mg) vitamin E; 18.2 mg vitamin C/100 g product.

### Example 8

### Soy functional food basic material with a composition beneficial for overweight or obese patients

In every aspect Example 7 is followed, except that for 200 g of germinated soy (YASO) by a standard method 300 ml aqueous solution of magnesium chloride x 6H₂O (4.16 g), calcium chloride x 2H₂O (1.8 g), 70 µg sodium selenite, 200 µg folic acid, furthermore 500 mg L-carnitine and 20 mg coenzyme Q₁₀ is used as cooking solution, in nitrogen atmosphere.

The incorporated bioactive material concentration of the product is as follows: 181 mg (77.3 mg) Mg; 232 mg (99.3 mg) Ca; 17 µg selenium; 249 µg folic acid; 127.4 mg L-carnitine and 6.7 mg coenzyme Q₁₀/100 g product.

### Example 9

### Functional food basic material beneficial for patients suffering from osteoporosis

In every aspect Example 7 is followed, except that for 200 g of germinated soy (YASO) by a standard method 300 ml aqueous solution of magnesium chloride x 6H₂O (1.00 g), calcium chloride x 2H₂O (0.12 g), zinc sulphate x 7H₂O, 0.04 g manganese sulphate x 2H₂O, and 0.05 g copper sulphate x 5 H₂O is used as cooking solution in atmospheric pressure and at a temperature of 100 °C.

The incorporated bioactive material concentration of the product is as follows: 121.34 mg (72.12 mg) Mg; 247.05 mg (98.3 mg) Ca; 5.80 mg (1.97 mg) Zn; 1.45 mg (0.78 mg) Mn; 1.12 mg (0.63 mg) Cu/100 g product.

### Example 10

### Functional food basic material beneficial for patients suffering from digestive and gastrointestinal problems

In every aspect Example 7 is followed, except that as cooking solution the solution of 650 mg thiamine (vitamin B1), 5 g niacin (vitamin B3) and 2,2 g ascorbic acid is used.

The incorporated bioactive material concentration of the soy product germinated for a short period of time according to the example is as follows: 1.2 mg (0.055 mg) thiamine; 14.2 mg (0.051 mg) niacin and 20 mg ascorbic acid /100 g product.

### Example 11

In every aspect Example 7 is followed except that rice or wheat or rye or oat or maize is germinated instead of soy, and the solutions according to Example 8 or 9 or 10 or 11 or 12 or 13 are used.

## Claims

1. A process for the preparation of raw materials of functional food, **characterised in that** a) the enzymes of the edible plant seeds are activated at a temperature of 10-35 °C for a period of 5-72 hours with oxygen and water until reaching the maximum nutritional value of the seeds, then b) the seeds thus obtained together with their sprouts are treated in the aqueous solution or suspension or emulsion of material selected from the group of ions, trace elements, vitamins, poly-unsaturated fatty acids, L-carnitine, coenzyme Q10, antioxidants, lecithin, gamma orisanol, folic acid, gamma amino butyric acid and koline, between 60-100 °C for a period of 10-180 minutes, and then the thus obtained raw material containing said biological active materials in enhanced concentration is separated and dried.

2. The process as claimed in Claim 1, **characterised in that** as edible plant seeds the seeds of cereals, leguminous plants are used.

3. The process as claimed in Claim 1 or 2, **characterised in that** as edible plant seeds the seeds of soy-bean, rice, wheat, oat, chickpea or maize, or their germinated forms generated by a method known per se is used.

4. Raw materials of functional food enriched in active compounds **characterised in that** it is prepared by any process as claimed in Claims 1 to 3.

5. Use of the product as claimed in Claim 4 for the preparation of functional foods.

## Patentansprüche

1. Verfahren zur Herstellung von Rohmaterialien für funktionelle Lebensmittel, **dadurch gekennzeichnet, dass** a) die Enzyme der essbaren Pflanzensamen bei einer Temperatur von 10 bis 35°C für einen Zeitraum von 5 bis 72 Stunden mit Sauerstoff und Wasser aktiviert werden, bis der maximale Nährwert der Samen erreicht ist, dann b) die so erhaltenen Samen zusammen mit ihren Keimen in der wässrigen Lösung oder Suspension oder Emulsion von Materialien, ausgewählt aus der Gruppe, bestehend aus Ionen, Spurenelementen, Vitaminen, polyungesättigten Fettsäuren, L-Carnitin, Coenzym Q10, Antioxidantien, Lecithin, gamma-Orisanol, Folsäure, gamma-Aminobuttersäure und Cholin, zwischen 60-100°C für einen Zeitraum von 10-180 Minuten behandelt werden, und dann das so erhaltene Rohmaterial, das die biologischen aktiven Materialien in erhöhter Konzentration enthält, getrennt und getrocknet wird.

2. Verfahren nach Anspruch 1, **dadurch gekennzeichnet, dass** als essbare Pflanzensamen die Samen von Getreide und Leguminosen verwendet werden.

3. Verfahren nach Anspruch 1 oder 2, **dadurch gekennzeichnet, dass** als essbare Pflanzensamen die Samen von Sojabohne, Reis, Weizen, Hafer, Kichererbse oder Mais oder deren gekeimte Formen verwendet werden, die durch ein an sich bekanntes Verfahren erzeugt werden.

4. Rohmaterialien für funktionelle Lebensmittel, die an Wirkstoffen angereichert sind, **dadurch gekennzeichnet, dass** sie gemäß einem Verfahren nach den Ansprüchen 1 bis 3 hergestellt wurden.

5. Verwendung des Produkts nach Anspruch 4 zur Herstellung funktioneller Lebensmittel.

## Revendications

1. Procédé pour la préparation de matières premières d'aliments fonctionnels, **caractérisé en ce que** a) les enzymes des graines de plantes comestibles sont activées à une température de 10-35°C pour une période de 5-72 heures avec de l'oxygène et de l'eau jusqu'à atteindre la valeur nutritionnelle maximale des graines, puis b) les graines ainsi obtenues conjointement avec leurs germes sont traitées dans la solution ou suspension ou émulsion aqueuse de matière sélectionnée parmi le groupe des ions, des oligo-éléments, des vitamines, des acides gras polyinsaturés, de la L-carnitine, de la coenzyme Q10, des antioxydants, de la lécithine, du gamma oryzanol, de l'acide folique, de l'acide gamma-aminobutyrique et de la choline, entre 60-100°C pour une période de 10-180 minutes, et ensuite la matière première ainsi obtenue contenant lesdites matières actives biologiques dans une concentration améliorée est séparée et séchée.

2. Le procédé selon la revendication 1, **caractérisé en ce qu'**en tant que graines de plantes comestibles les graines de céréales, de plantes légumineuses sont utilisées.

3. Le procédé selon la revendication 1 ou 2, **caractérisé en ce qu'**en tant que graines de plantes comestibles les graines de soja, de riz, de blé, d'avoine, de pois chiches ou de maïs ou leurs formes germées générées par un procédé connu en soi sont utilisées.

4. Matières premières d'aliments fonctionnels enrichies en composés actifs **caractérisées en ce qu'**elles sont préparées par un quelconque procédé tel que revendiqué dans les revendications 1 à 3.

5. Utilisation du produit tel que revendiqué dans la revendication 4 pour la préparation d'aliments fonctionnels.
